# EUROPÄISCHE PATENTSCHRIFT

(11) **EP 1 711 215 B1**
(45) Veröffentlichungstag und Bekanntmachung des Hinweises auf die Patenterteilung: **13.03.2013**
(21) Anmeldenummer: 05706716.7
(22) Anmeldetag: 27.01.2005
(51) Int. Cl.: A61M 1/28, A61M 39/22, A61M 39/20

(54) **VORRICHTUNG UND VERFAHREN ZUM EINBRINGEN EINES VERSCHLUSSSTOPFENS IN EINEN PATIENTENKONNEKTOR FÜR DIE PERITONEALDIALYSE**
DEVICE AND METHOD FOR INTRODUCING A PLUG INTO THE PERITONEAL DIALYSIS CONNECTOR OF A PATIENT
DISPOSITIF ET PROCEDE D'INTRODUCTION D'UN BOUCHON DE FERMETURE DANS UN CONNECTEUR DE DIALYSE PERITONEALE SITUE DANS LE PATIENT

(30) Priorität: 03.02.2004 DE 102004005372
(43) Veröffentlichungstag der Anmeldung: 18.10.2006
(73) Patentinhaber: Fresenius Medical Care Deutschland GmbH, 61352 Bad Homburg (DE)
(72) Erfinder: BIESEL, Wolfgang, 66564 Ottweiler (DE); REITER, Reinhold, I-26013 Crema (IT); VENERONI, Alain, I-26016 Spino d'Adda (IT)
(74) Vertreter: Wieske, Thilo
(86) Internationale Anmeldenummer: PCT/DE2005/000126
(87) Internationale Veröffentlichungsnummer: WO 2005/075008

(56) Entgegenhaltungen:
- EP-A- 0 198 407
- WO-A-2004/011077
- DE-C1- 19 814 047
- US-A- 3 707 972
- US-A- 5 228 646

## Beschreibung

Die Erfindung betrifft eine Vorrichtung und ein Verfahren zum Einbringen eines Verschlußstopfens in einen Patientenkonnektor für die Peritonealdialyse.

Patientenkonnektoren für die Peritonealdialyse sind beispielsweise aus der EP 0 715 860 B1 oder der DE 198 14 047 C1 bekannt. Diese ermöglichten eine sterile Konnektion und Dekonnektion im Rahmen einer Peritonealdialyse-Behandlung. Nach Beendigung der Peritonealdialyse wird mit derartigen Patientenkonnektoren ein Verschlußstopfen in den Konnektor des Schlauchstückes, das in die Bauchhöhle des Patienten geht, eingebracht. Somit ist der Zugang zur Bauchhöhle des Patienten steril verschlossen.

Wenn der Patient sich von seinem Schlauchset dekonnektieren und später wieder konnektieren möchte, so ist dies grundsätzlich möglich, wenn nicht der Verschlußstopfen bereits verbraucht worden ist. Ein zwei- oder mehrfaches Dekonnektieren und wieder Konnektieren ist daher nicht möglich. Bislang wird diesem Problem dadurch begegnet, daß zwei Patientenkonnektoren mit jeweils einem Verschlußstopfen durch ein kurzes Schlauchstück getrennt hintereinander an einem Schlauchset angeordnet sind. Dadurch wird es möglich, sich im Verlauf der Behandlung einmal zu dekonnektieren und wieder zu konnektieren. Der bei dem ersten Dekonnektionsvorgang "verbrauchte" Patientenkonnektor wird vor der erneuten Konnektion einfach entsorgt. Der zusätzliche Patientenkonnektor inklusive des kurzen Schlauchstücks ist - insbesondere in Anbetracht der Tatsache, daß der zweifache Patientenkonnektor nur in etwa 10 % der Fälle benötigt wird - jedoch relativ teuer und es kann insgesamt mit dem selben Schlauchset nur ein Dekonnektier-/Konnektiervorgang durchgeführt werden.

Die US 3,707,972 A beschreibt einen Konnektor für ein Drainagesystem, beispielsweise für die Blase. Es wird das Verschließen eines Flüssigkeitsdurchgangs in dem Konnektor durch Andrücken eines Verschlußstopfens an einen entsprechend ausgeformten Sitz beschrieben. Aus der nachveröffentlichten WO 2004/011077 A1 ist ein steriler Konnektor bekannt, der auch zum Herstellen einer Verbindung zwischen einem Dialysebeutel und einem Patienten verwendet werden kann.

Aufgabe der Erfindung ist es somit, bei Patientenkonnektoren, die bei dem Dekonnektionsvorgang einen sterilen Verschlußstopfen in den Bauchanschluß des Patienten einbringen, eine beliebige Zahl von Dekonnektier-/Konnektiervorgängen zu ermöglichen, wobei die damit verbundenen Kosten möglichst gering sein sollten.

Diese Aufgabe wird erfindungsgemäß durch eine Vorrichtung zum Einbringen eines Verschlußstopfens in einen Patientenkonnektor für die Peritonealdialyse, dessen Verschlußstopfen bereits verwendet worden ist, bestehend aus einem Gehäuse, in dem ein Verschlußstopfen vor dem Übertragen kontaminationsgeschützt in einer zurückgesetzten Position in dem Gehäuse angeordnet ist und welche mit dem Bereich des Patientenkonnektors, in den der Vershlußstopfen übertragen werden soll, verbindbar ist und aus Mitteln zum Übertragen des Verschlußstopfens aus der Vorrichtung in den Patientenkonnektor, gelöst.

Mit einer derartigen Vorrichtung kann in einen Patientenkonnektor, dessen Verschlußstopfen bereits verwendet worden ist, unter sterilen Bedingungen ein neuer Verschlußstopfen eingebracht werden. Bei einem Verschlußstopfen wie dem aus der DE 198 14 047 C1 wird durch das Einbringen eines neuen Verschlußstopfens auch der Druckknopf, mit dem der Verschlußstopfen eingebracht wird, wieder in die Ausgangsstellung zurückgeführt, so daß der Patientenkonnektor wieder für einen Konnektions-/Dekonnektionsvorgang zur Verfügung steht. Auch eine mehrfache Wiederbeladung eines Patientenkonnektors mit Verschlußstopfen ist auf diese Weise möglich. Diese Vorgehensweise ist insofern vorteilhaft, als nur bei einem tatsächlich beabsichtigten Konnektions-/Dekonnektionsvorgang Kosten anfallen. Durch die Verbindung von Gehäuse und Patientenkonnektor wird ein einfaches Übertragen des Verbindungsstopfens unter sterilen Bedingungen ermöglicht. Ein Bedienungsfehler kann hierdurch praktisch ausgeschlossen werden, so daß weder ein Verlust der Sterilität des Verschlußstopfens oder dessen Verlust bei der Übertragung zu befürchten sind. Durch die zurückgesetzte Position des Verschlußstopfens wird verhindert, daß dieser von dem Benutzer berührt und hierbei kontaminiert werden kann.

Hierbei kann vorgesehen sein, daß das Gehäuse durch Formschluß, insbesondere durch eine Schraub-, Stift- oder Keilverbindung, mit dem Patientenkonnektor verbindbar ist.

Ebenso ist es möglich, daß das Gehäuse durch Kraftschluß, insbesondere durch eine Preß- oder Klemmverbindung, mit dem Patientenkonnektor verbindbar ist.

Weiterhin ist es zweckmäßig, daß die Mittel zum Übertragen des Verschlußstopfens als ein Druck- oder Drehknopf an dem der Austrittsstelle des Verschlußstopfens gegenüberliegenden Ende des Gehäuses sowie gegebenenfalls ein Zwischenelement zwischen dem Druck- bzw. Drehknopf und dem Verschlußstopfen ausgebildet sind.

Durch Betätigung des Druck- oder Drehknopfes wird der Verschlußstopfen linear innerhalb des Gehäuses verschoben und schließlich aus dem Gehäuse hinaus in die hierfür vorgesehene Öffnung des Patientenkonnektors hinein verschoben. Gegebenenfalls kann ein Zwischenelement zwischen dem Druck- oder Drehknopf und dem Verschlußstopfen angeordnet sein.

Erfindungsgemäß ist auch, daß die Mittel zum Übertragen des Verschlußstopfens als eine Halterung des Verschlußstopfens ausgebildet sind, deren Haltekraft auf den Verschlußstopfen geringer ist als die Haltekraft des Verschlußstopfens in dem Patientenkonnektor.

In einer derartigen Halterung plaziert wird der Verschlußstopfen in die Öffnung des Patientenkonnektors eingeführt, löst sich aufgrund der größeren Haltekraft aus dem Gehäuse und wird einsatzbereit in dem Patientenkonnektor gehalten.

Im Rahmen der Erfindung liegt auch ein Verfahren zum Einbringen eines Verschlußstopfens in einen Patientenkonnektor für die Peritonealdialyse, wobei ein Gehäuse, in dem ein Verschlußstopfen angeordnet ist, mit dem Bereich eines Patientenkonnektors, in den der Verschlußstopfen übertragen werden soll, verbunden wird und anschließend der Verschlußstopfen von dem Gehäuse in den Patientenkonnektor übertragen wird.

Hierbei ist es zweckmäßig, daß das Gehäuse durch Formschluß oder durch Kraftschluß mit dem Patientenkonnektor verbunden wird.

Vorteilhaft ist auch, daß der Verschlußstopfen durch lineare Verschiebung von dem Gehäuse in den Patientenkonnektor übertragen wird.

Ebenso ist erfindungsgemäß vorgesehen, daß die lineare Verschiebung des Verschlußstopfens durch Betätigung eines Druck- oder Drehknopfes ausgelöst wird.

Schließlich ist auch zur Erfindung gehörig, daß der Verschlußstopfen dadurch übertragen wird, daß die Haltekraft der Halterung des Verschlußstopfens in dem Gehäuse auf den Verschlußstopfen geringer ist als die Haltekraft des Verschlußstopfens in dem Patientenkonnelctor.

Nachfolgend werden Ausführungsbeispiele der Erfindung anhand von Zeichnungen beschrieben.

Es zeigen
- Fig. 1: eine perspektivische Darstellung einer erfindungsgemäßen Vorrichtung,
- Fig. 2 a und Fig. 2b: in geschnittener Darstellung den Wiederbeladevorgang eines Patientenkonnektors mit Hilfe der in Fig. 1 dargestellten Vorrichtung,
- Fig. 3: eine perspektivische Darstellung einer anderen Ausführung der Vorrichtung,
- Fig. 4a bis Fig. 4i: die Darstellung eines Wiederbeladevorganges eines Patientenkonnektors unter Verwendung eines sogenannten "Organizers,
- Fig. 5a bis Fig. 5e: in teilgeschnittener und geschnittener Darstellung den Wiederbeladevorgang mit einer Vorrichtung zum Verschieben des Verschlußstopfens,
- Fig. 6a bis Fig. 6c: in geschnittener Darstellung den Wiederbeladevorgang mit einer Vorrichtung zum Übertragen des Verschlußstopfens durch eine Schraubbewegung.

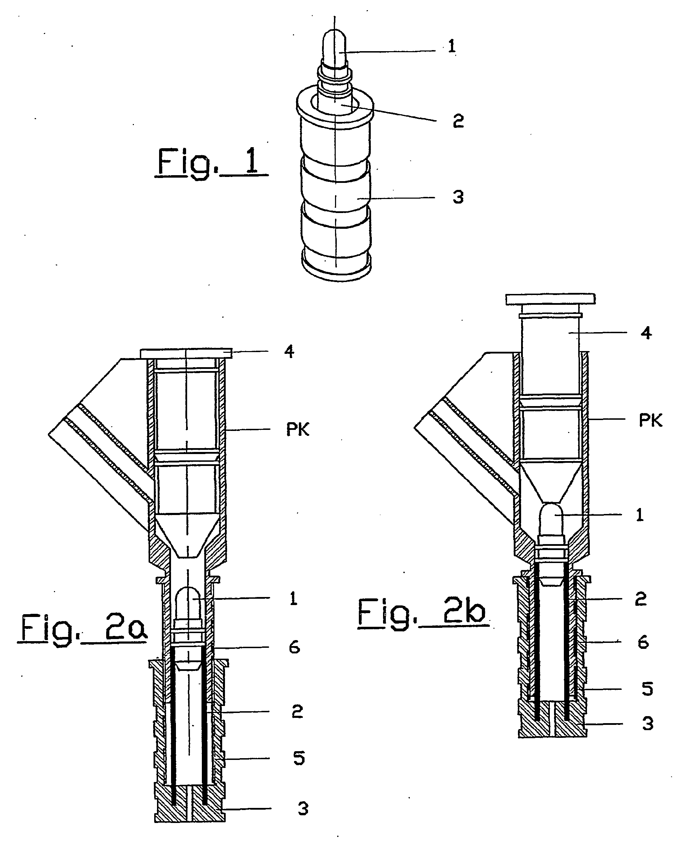

Wie aus Fig. 1 ersichtlich, ist eine einfache Ausbildung der erfindungsgemäßen Vorrichtung derart möglich, daß ein steriler Verschlußstopfen 1 auf einem zylindrischen Rohrabschnitt 2 angeordnet ist, welcher am Boden einer Schutzkappe 3 befestigt ist. Die Schutzkappe 3 dient der Handhabung der Vorrichtung, so daß weder der Verschlußstopfen 1 noch der Rohrabschnitt 2 während des Wiederbeladevorganges des Patientenkonnektors (PK) berührt werden müssen.

Fig. 2a zeigt den Beginn des Wiederbeladevorgangs bei einem Patientenkonnektor (PK), wie er aus der DE 198 14 047 C1 bekannt ist: Der Druckknopf 4 des Patientenkonnektors (PK) ist durch das vorherige Einbringen eines Verschlußstopfens noch in abgesenkter Stellung. In das dem Druckknopf 4 gegenüberliegende, stutzenförmig ausgebildete Ende des Patientenkonnektors (PK) wird der den neuen, sterilen Verschlußstopfen 1 tragende Rohrabschnitt 2 eingeführt, bis der neue Verschlußstopfen 1 an dem unteren Ende des Druckknopfs 4 anliegt. Anschließend (Fig. 2b) wird das Innengewinde 5 der Schutzkappe 3 auf das Außengewinde 6 des Patientenkonnekrtors (PK) geschraubt, wodurch der Verschlußstopfen 1 Druck auf den Druckknopf 4 des Patientenkonnektors (PK) ausübt und diesen wieder in seine Ausgangsstellung nach oben zurückbewegt. Fig. 2b zeigt die Endstellung des Wiederbeladevorgangs: Der Druckknopf 4 befindet sich erneut in der Ausgangsstellung und kann Druck auf den unmittelbar davor in dem Stutzen des Patientenkonnektors (PK) angeordneten Verschlußstopfen 1 ausüben, so daß mit dem Patientenkonnektor (PK) ein weiterer Konnektions-/Dekonnektionsvorgang durchgeführt werden kann. Aufgrund der Tatsache, daß die Haltekraft zwischen dem Patientenkonnektor (PK) und dem Verschlußstopfen 1 größer ist als diejenige zwischen dem Rohrabschnitt 2 und dem Verschlußstopfen 1 verbleibt der Verschlußstopfen 1 bei Entfernen der Schutzkappe 3 sowie des daran angeordneten Rohrabschnitts 2 aus dem Patientenkonnektor (PK) in dem letzteren.

Der zu Beginn des Wiederaufladevorgangs sterile Verschlußstopfen (die gesamte Wiederaufladeeinheit ist zweckmäßigerweise steril verpackt) wird während des Wiederaufladevorgangs nicht berührt. Dies kann dadurch erreicht werden, daß der Verschlußstopfen 1 zunächst in einer zurückgesetzten Position in dem Gehäuse (7) sitzt, so daß ein Berührschutz gegen Kontamination gegeben ist.

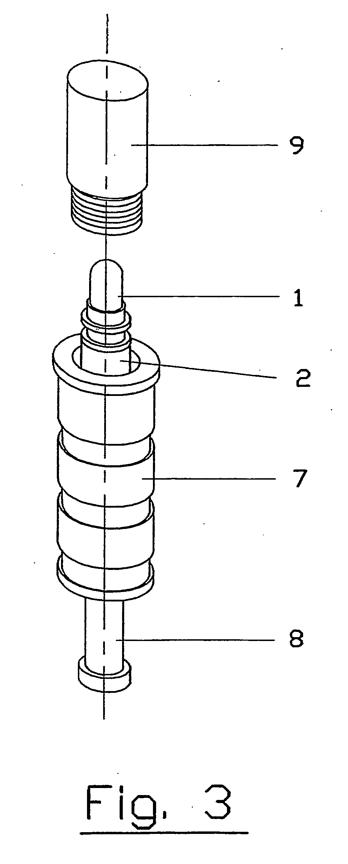

Fig. 3 zeigt eine andere Ausbildung einer erfindungsgemäßen Vorrichtung. Bei dieser Ausbildung ist der Verschlußstopfen 1 innerhalb eines zylindrischen Gehäuses 7 ebenfalls auf einem Rohrabschnitt 2 mit zylindrischem Querschnitt angeordnet, wobei der Rohrabschnitt 2 innerhalb des Gehäuses 7 linear verschiebbar ist.

Das Einbringen des Verschlußstopfens 1 in einen Patientenkonnektor (PK) erfolgt analog dem oben beschriebenen Vorgang, wobei die lineare Verschiebung des Verschlußstopfens 1 durch die Betätigung des Betätigungsknopfes 8 - eventuell unter Zwischenschaltung eines Zwischenelementes zwischen dem Betätigungsknopf 8 und dem Rohrabschnitt 2 ausgelöst wird.

In Fig. 3 ist zur besseren Sichtbarkeit des Verschlußstopfens 1 vor dem Wiederbeladevorgang (Betätigungsknopf 8 noch nicht betätigt) in aus dem Gehäuse 7 ausgeschobener Stellung dargestellt. Vor dem Einbringen in den Patientenkonnektor (PK) ist der Verschlußstopfen innerhalb des Gehäuses 7 steril und vor Kontamination geschützt untergebracht und kann zudem durch die ebenfalls dargestellte Kappe 9 geschützt sein. Darüber hinaus bietet sich das Einschweißen der Vorrichtung im sterilen Zustand zur dauerhaften Aufbewahrung an.

Der Wiederaufladevorgang wird nachfolgend beschrieben, wobei dieser unter Verwendung eines sogenannten "Organizers", einer Vorrichtung zur Aufnahme des Patientenkonnektors (PK), der die Handhabung seitens des Patienten deutlich erleichtert, dargestellt ist.

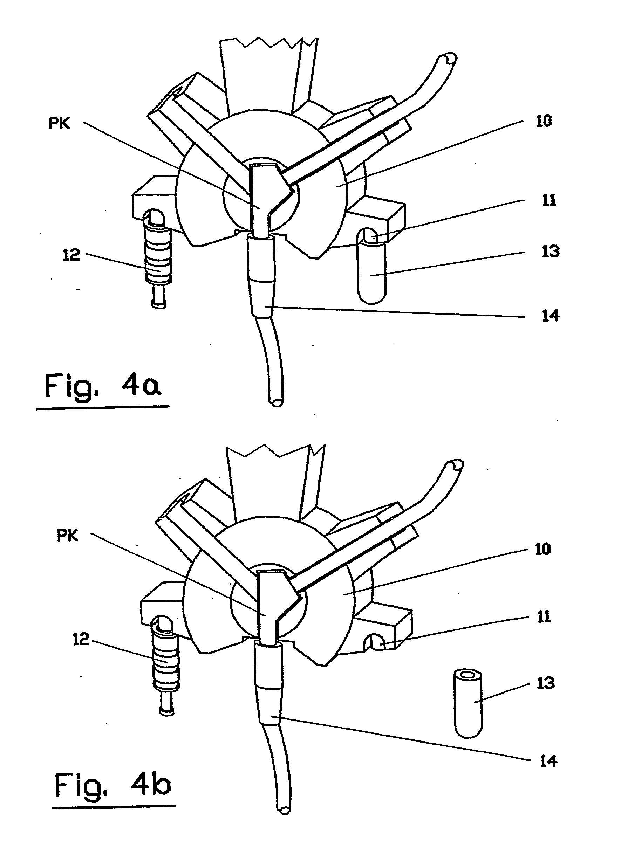

Wie in Fig. 4a dargestellt, wird der Patientenkonnektor (PK), hier der aus der DE 198 14 047 C1 bekannte, in den Zentralbereich des Organizers 10 eingelegt. Weiterhin sind eine Desinfektionskappe 11 und die Wiederbeladevorrichtung 12, wie sie aus Fig. 3 bekannt ist, an dem Organizer 10 angeordnet.

Der Organizer 10 wird weiterhin für den Dekonnektionsvorgang vorbereitet, indem, wie in Fig. 4b gezeigt, eine Schutzkappe 13 von der Desinfektionskappe 11 entfernt wird.

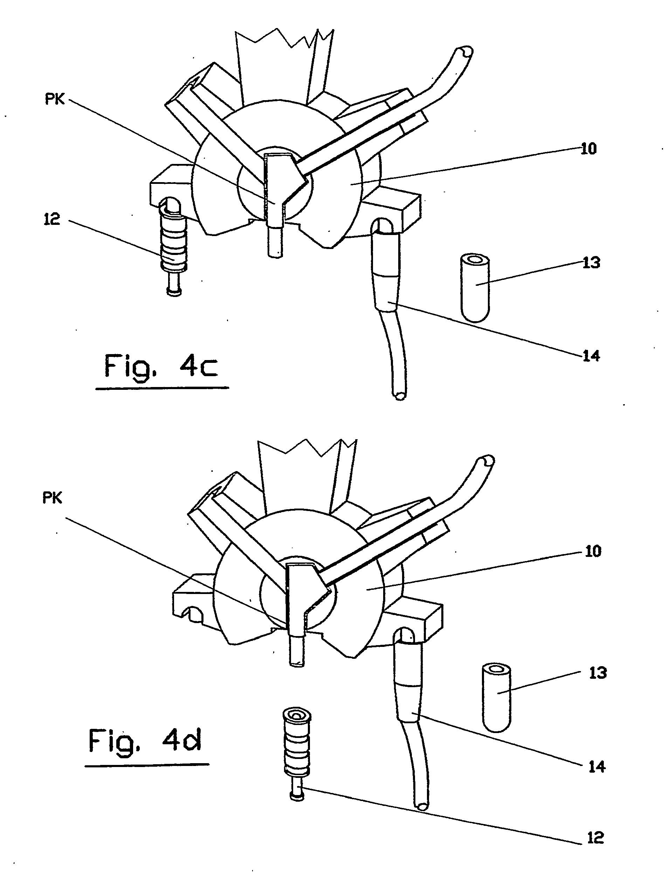

Nach Beendigung dieser Vorbereitungen kann der Dekonnektionsvorgang erfolgen. Hierfür wird, wie in Fig. 4c gezeigt, zunächst der mit einem Verschlußstopfen 1 verschlossene Bauchanschluß 14 von dem Patientenkonnektor PK gelöst und mit der Desinfektionskappe 11 verbunden.

Dann wird (Fig. 4d) die Wiederbeladevorrichtung 12 von dem Organizer 10 gelöst. In ihr befindet sich ein neuer steriler Verschlußstopfen 1, der dort vor Kontamination geschützt ist. Die Kappe 9 verbleibt hierbei am Organizer 10.

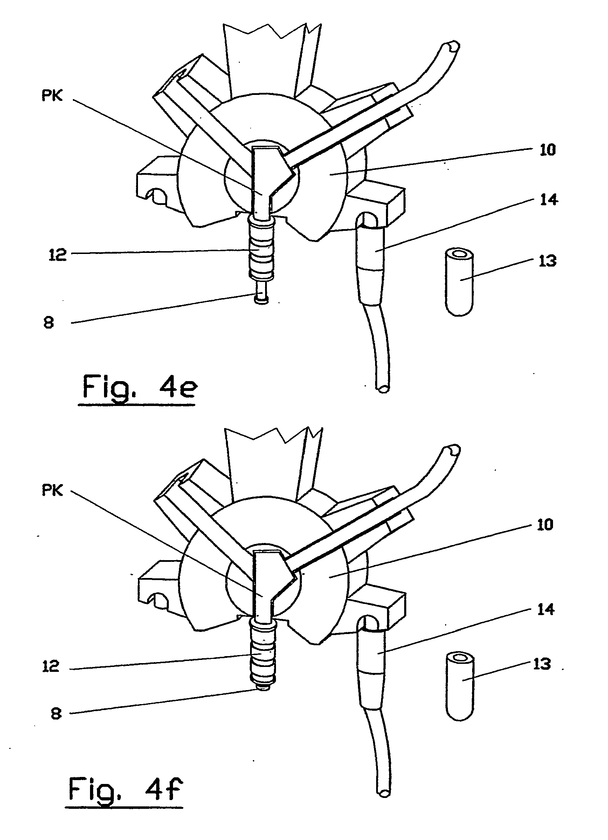

Nun wird die Wiederbeladevorrichtung 12 - mit dem in dem Organizer 10 angeordneten Patientenkonnektor PK verbunden (Fig. 4e) und der Betätigungsknopf 8 der Wiederbeladevorrichtung 12 gedrückt, um den neuen Verschlußstopfen 1 in den Patientenkonnektor PK zu übertragen und dessen Druckknopf 4 wieder in die Ausgangsstellung zurückzuführen (Fig. 4f).

Der Patientenkonnektor PK ist nun bereit für einen neuen Konnektionsvorgang.

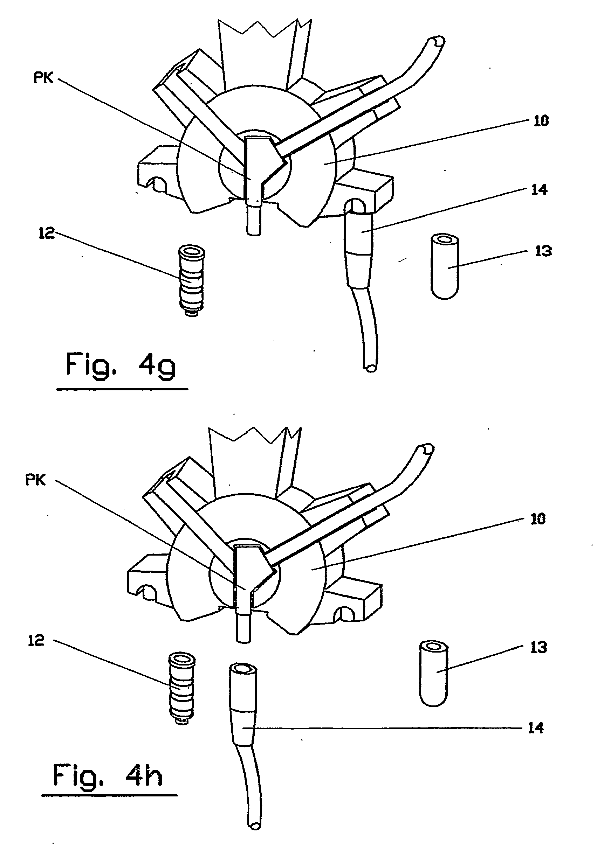

Hierfür wird zunächst die um ihren Verschlußstopfen 1 entleerte Wiederbeladevorrichtung 12 von dem Patientenkonnektor PK entfernt, wie in Fig. 4g dargestellt.

Fig. 4h zeigt, wie dann der Bauchanschluß 14 (ohne Verschlußstopfen 1, der in der Desinfektionskappe11 verbleibt, die in dekonnektiertem Zustand auf dem Bauchanschluß 14 sitzt) des Patienten wieder mit dem Patientenkonnektor (PK) verbunden wird.

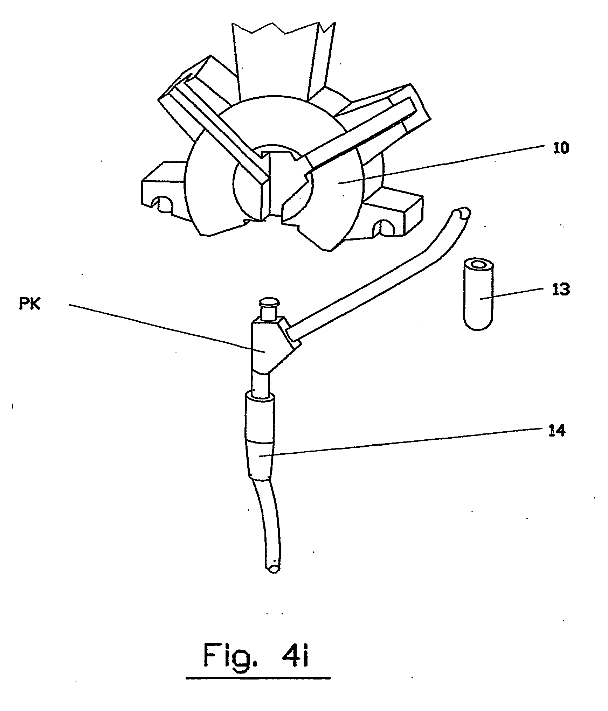

Nun (Fig. 4i) ist der Patientenkonnektor PK wieder mit einem Verschlußstopfen 1 versehen und der Druckknopf 4 kann erneut betätigt werden, um mit dem Verschlußstopfen 1 den nächsten Dekonnektionsvorgang durchzuführen.

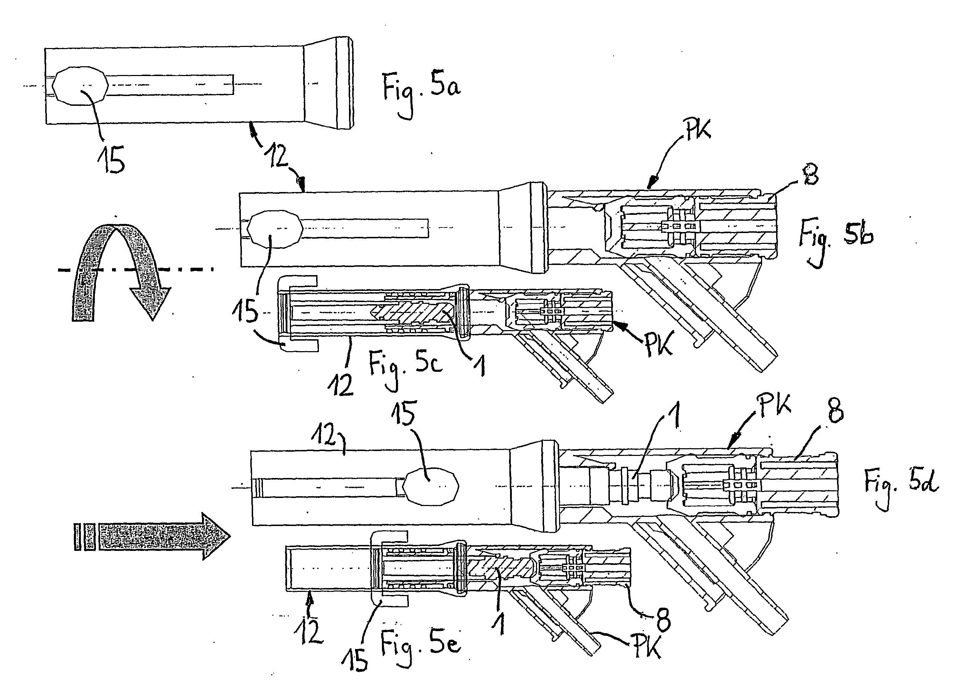

Fig. 5a bis 5e zeigen den Aufbau und die Anwendung einer Wiederbeladevorrichtung 12 zum Wiederbeladen eines Patientenkonnektors PK mit einem Verschlußstopfen 1, bei der zur Übertragung des Verschlußstopfens 1 eine verschiebbare Betätigungsrichtung 15 vorgesehen ist. Diese ist in den Fig. 5a bis 5c in ihrer Ausgangsposition dargestellt. Die Wiederbeladevorrichtung 12 wird in einem ersten Schritt (Fig. 5b und 5c) an das freie Ende des Patientenkonnektors PK angeschraubt. Anschließend wird in einem zweiten Schritt (Fig. 5d und Fig. 5e) durch Verschieben der Betätigungsvorrichtung 15, die als von Hand betätigbarer Schieber ausgebildet ist, der Verschlußstopfen 1 aus der geschützten Stellung in der Wiederbeladevorrichtung 12 in den Patientenkonnektor PK verschoben, wobei der Betätigungsknopf 8 des Patientenkonnektors PK wieder in die ausgefahrene Stellung, welche eine erneute Betätigung ermöglicht, zurückgeführt wird.

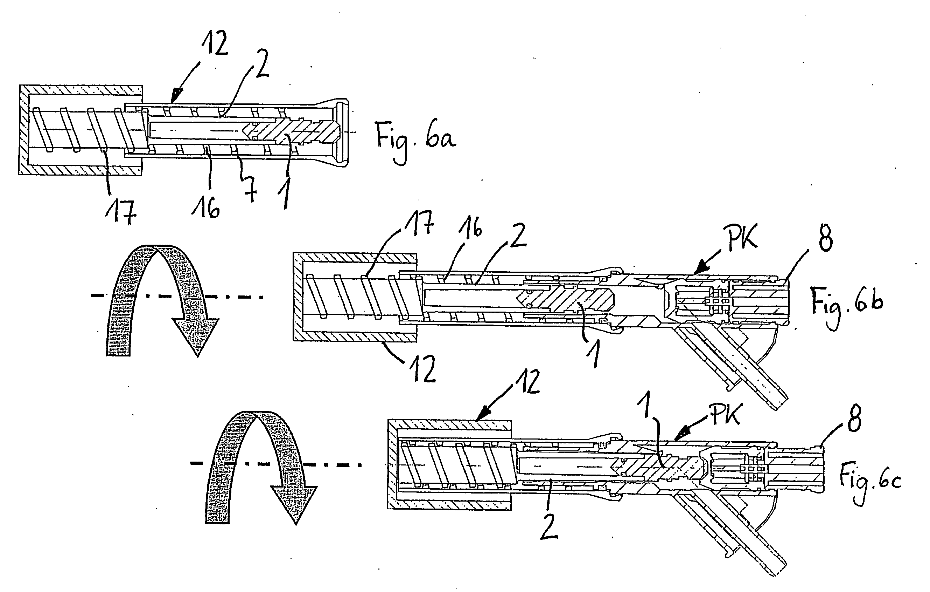

Fig. 6a bis Fig. 6c zeigen eine weitere Alternative einer solchen Wiederbeladevorrichtung 12, die in einem ersten Schritt (Fig. 6b) auf das freie Ende des Patientenkonnektors PK aufgeschraubt wird. Das Gehäuse 7, das den Rohrabschnitt 2, welcher den Verschlußstopfen 1 trägt, schützend umgibt ist bei dieser Ausgestaltung mit einem Innengewinde 16 versehen. Der Rohrabschnitt 2 weist ein entsprechendes Außengewinde 17 auf. Durch das in Fig. 6c dargestellte Verschrauben des hinteren, den Rohrabschnitt 2 tragenden Bereichs der Wiederbeladevorrichtung 12 mit dem Gehäuse 7 wird der Verschlußstopfen 1 in letzteren eingeschoben und der Betätigungsknopf 8 wieder in die Ausgangsstellung zurückgeführt.

## Patentansprüche

1. Vorrichtung (12) zum Übertragen eines neuen Verschlußstopfens (1) in einen Patientenkonnektor (PK) für die Peritonealdialyse, dessen Verschlußstopfen bereits verwendet worden ist, bestehend aus einem Gehäuse (3, 7), in dem ein Verschlußstopfen (1) vor dem Übertragen kontaminationsgeschützt in einer zurückgesetzten Position in dem Gehäuse (3, 7) angeordnet ist und welches mit dem Bereich des Patientenkonnektors, in den der Verschlußstopfen übertragen werden soll, verbindbar ist und aus Mitteln (2, 8) zum Übertragen des Verschlußstopfens (1) aus der Vorrichtung (12) in den Patientenkonnektor (PK).

2. Vorrichtung gemäß Anspruch 1, **dadurch gekennzeichnet, daß** das Gehäuse (3, 7) durch Formschluß, insbesondere durch eine Schraub-, Stift- oder Keilverbindung, mit dem Patientenkonnektor (PK) verbindbar ist.

3. Vorrichtung gemäß Anspruch 1, **dadurch gekennzeichnet, daß** das Gehäuse (3, 7) durch Kraftschluß, insbesondere durch eine Preß- oder Klemmverbindung, mit dem Patientenkonnektor (PK) verbindbar ist.

4. Vorrichtung gemäß Anspruch 1, **dadurch gekennzeichnet, daß** die Mittel (8) zum Übertragen des Verschlußstopfens als ein Druck- oder Drehknopf (8) an dem der Austrittsstelle des Verschlußstopfens (1) gegenüberliegenden Ende des Gehäuses (7) sowie gegebenenfalls ein Zwischenelement zwischen dem Druck- bzw. Drehknopf (8) und dem Verschlußstopfen (1) ausgebildet sind.

5. Vorrichtung gemäß Anspruch 1, **dadurch gekennzeichnet, daß** die Mittel (2) zum Übertragen des Verschlußstopfens (1) als eine Halterung (2) des Verschlußstopfens (1) ausgebildet sind, deren Haltekraft auf den Verschlußstopfen (1) geringer ist als die Haltekraft des Verschlußstopfens (1) in dem Patientenkonnektor (PK).

6. Verfahren zum Einbringen eines Verschlußstopfens (1) in einen Patientenkonnektor (PK) für die Peritonealdialyse mit einer Vorrichtung gemäß den Ansprüche 1 bis 5, **dadurch gekennzeichnet, daß** das Gehäuse (3, 7), in dem der Verschlußstopfen (1) angeordnet ist, mit dem Bereich eines Patientenkonnektors (PK), in den der Verschlußstopfen (1) übertragen werden soll, verbunden wird und daß anschließend der Verschlußstopfen (1) von dem Gehäuse (3, 7) in den Patientenkonnektor (PK) übertragen wird.

7. Verfahren gemäß Anspruch 6, **dadurch gekennzeichnet, daß** das Gehäuse (3, 7) durch Formschluß oder durch Kraftschluß mit dem Patientenkonnektor (PK) verbunden wird.

8. Verfahren gemäß einem der Ansprüche 6 oder 7, **dadurch gekennzeichnet, daß** der Verschlußstopfen (1) durch lineare Verschiebung von dem Gehäuse (3, 7) in den Patientenkonnektor (PK) übertragen wird.

9. Verfahren gemäß einem der Ansprüche 6 bis 8, **dadurch gekennzeichnet, daß** die lineare Verschiebung des Verschlußstopfens (1) durch Betätigung eines Druck- oder Drehknopfes (8) ausgelöst wird.

10. Verfahren gemäß einem der Ansprüche 6 bis 8, **dadurch gekennzeichnet, daß** der Verschlußstopfen (1) dadurch übertragen wird, daß die Haltekraft der Halterung des Verschlußstopfens (1) in dem Gehäuse (3, 7) auf den Verschlußstopfen (1) geringer ist als die Haltekraft des Verschlußstopfens(1) in dem Patientenkonnektor (PK).

## Claims

1. A device (12) for transferring a new closure plug (1) into a patient connector (PK) for peritoneal dialysis, the closure plug in said connector having already been used, said device consisting of a housing (3, 7) containing a closure plug (1) which, prior to transfer, is held, protected from contamination, in a retracted position within the housing (3,7), the housing being connectable with the portion of the patient connector into which the closure plug is to be transferred, and of means (2,8) for transferring the closure plug (1) from the device (12) to the patient connector (PK).

2. A device according to claim 1, **characterised in that** the housing (3, 7) is connectable with the patent connector (PK) by means of a form fit, in particular a screwed, pin-type or keyed joint.

3. A device according to claim 1, **characterised in that** the housing (3, 7) is connectable with the patent connector (PK) by means of a frictional connection, in particular a press fit or a clamped joint.

4. A device according to claim 1, **characterised in that** the means (8) for transferring the closure plug is engineered as a push-button or turning knob (8) at the opposite end of the housing (7) to where the closure plug (1) exits the same, with an intermediate element being provided if necessary between the push-button or turning knob (8) and the closure plug (1).

5. A device according to claim 1, **characterised in that** the means (2) for transferring the closure plug (1) is configured as a holder (2) for the closure plug (1), the retention force exerted by said holder on the closure plug (1) being lower than the retention force exerted on the closure plug (1) in the patient connector (PK).

6. A method of introducing a closure plug (1) into a patient connector (PK) for peritoneal dialysis with a device according to claims 1 to 5, **characterised in that** the housing (3, 7) containing the closure plug (1) is connected with the portion of a patient connector (PK) into which the closure plug (1) is to be transferred, and that the closure plug (1) is subsequently transfered from the housing (3, 7) to the patient connector (PK).

7. A method according to claim 6, **characterised in that** the housing (3, 7) is connectable with the patent connector (PK) by means of a form fit or a frictional connection.

8. A method according to one of claims 6 or 7, **characterised in that** the closure plug (1) is transferred by linear displacement from the housing (3, 7) into the patient connector (PK).

9. A method according to one of claims 6 to 8, **characterised in that** the linear displacement of the closure plug (1) is triggered by actuation of a push-button or turning knob (8).

10. A method according to one of claims 6 to 8, **characterised in that** the closure plug (1) is transferred as a result of the retention force exerted by the holder on the closure plug (1) in the housing (3, 7) being lower than the retention force exerted on the closure plug (1) in the patient connector (PK).

## Revendications

1. Dispositif (12) pour transférer un nouveau bouchon de fermeture (1) dans un connecteur de patient (PK) pour la dialyse péritonéale dont le bouchon de fermeture a déjà été utilisé, ledit dispositif étant constitué d'un boîtier (3, 7) contenant un bouchon de fermeture (1) qui est placé, avant le transfert, dans une position reculée dans le boîtier (3, 7) et protégé contre la contamination, lequel boîtier pouvant être connecté à la portion du connecteur de patient dans laquelle le bouchon de fermeture doit être transféré, et de moyens (2, 8) pour transférer le bouchon de fermeture (1) du dispositif (12) dans le connecteur de patient (PK).

2. Dispositif selon la revendication 1, **caractérisé en ce que** le boîtier (3, 7) peut être connecté au connecteur de patient (PK) par emboîtement de forme, notamment une liaison vissée, une liaison par baïonnette ou un clavetage.

3. Dispositif selon la revendication 1, **caractérisé en ce que** le boîtier (3, 7) peut être raccordé au connecteur de patient (PK) par une connexion par friction, notamment ajustement avec serrage ou un joint par serrage.

4. Dispositif selon la revendication 1, **caractérisé en ce que** les moyens (8) pour transférer le bouchon de fermeture sont conçus sous la forme d'un bouton poussoir ou d'un bouton tournant (8) disposé à l'extrémité du boîtier (7) opposée au point de sortie du bouchon de fermeture (1), ainsi que, le cas échéant, d'un élément intermédiaire disposé entre le bouton poussoir ou le bouton tournant (8) et le bouchon de fermeture (1).

5. Dispositif selon la revendication 1, **caractérisé en ce que** les moyens (2) pour transférer le bouchon de fermeture (1) sont conçus comme un support (2) pour le bouchon de fermeture (1) dont la force de retenue exercée sur le bouchon de fermeture (1) est inférieure à la force de retenue exercée sur le bouchon de fermeture (1) dans le connecteur de patient (PK).

6. Procédé pour introduire un bouchon de fermeture (1) dans un connecteur de patient (PK) pour une dialyse péritonéale avec un dispositif selon l'une des revendications 1 à 5, **caractérisé en ce que** le boîtier (3, 7) contenant le bouchon de fermeture (1) est raccordé avec la portion du connecteur de patient (PK) dans laquelle le bouchon de fermeture doit être transféré, et **en ce que** le bouchon de fermeture (1) est ensuite transféré du boîtier (3, 7) dans le connecteur de patient (PK).

7. Procédé selon la revendication 6, **caractérisé en ce que** le boîtier (3, 7) est raccordé au connecteur de patient (PK) par emboîtement de forme ou par une connexion par friction.

8. Procédé selon l'une des revendications 6 ou 7, **caractérisé en ce que** le bouchon de fermeture (1) est transféré du boîtier (3, 7) dans le connecteur de patient (PK) par un déplacement linéaire.

9. Procédé selon l'une des revendications 6 à 8, **caractérisé en ce que** le déplacement linéaire du bouchon de fermeture (1) est déclenché par l'actionnement d'un bouton poussoir ou un bouton tournant (8).

10. Procédé selon l'une des revendications 6 à 8, **caractérisé en ce que** le bouchon de fermeture (1) est transféré du fait que la force de retenue exercée sur le bouchon de fermeture par le support du bouchon de fermeture (1) dans le boîtier (3, 7) est inférieure à la force de retenue exercée sur le bouchon de fermeture (1) dans le connecteur de patient (PK).
